# EUROPEAN PATENT SPECIFICATION

(11) **EP 2 410 937 B1**
(45) Date of publication and mention of the grant of the patent: **06.01.2016**
(21) Application number: 09787669.2
(22) Date of filing: 23.03.2009
(51) Int. Cl.: A61C 5/00, A61K 6/00, A61K 6/027, A61K 6/08

(54) **SEMI -WORKED PIECE FOR PRODUCTION OF DENTAL/ODONTOIATRIC DEVICES, NAMELY FOR POSTS, STUMPS AND DENTAL CROWNS**
HALBZEUG ZUR HERSTELLUNG DENTALER ODER ODONTOIATRISCHER MITTEL ALS DENTALPFOSTEN, STÜMPFE ODER - KRONEN
PIÈCE SEMI-USINÉE POUR LA FABRICATION DE DISPOSITIFS À VOCATION DENTAIRE/ODONTOIATRIQUE, NOTAMMENT POUR DES TENONS RADICULAIRES, DES MOIGNONS ET DES COURONNES DENTAIRES

(43) Date of publication of application: 01.02.2012
(73) Proprietor: Bioloren S.R.L., 21047 Saronno (Varese) (IT)
(72) Inventor: RATTI, Umberto, I-21047 Saronno (Varese) (IT)
(74) Representative: Tarabbia, Luigi
(86) International application number: PCT/IT2009/000108
(87) International publication number: WO 2010/109496

(56) References cited:
- EP-A- 0 230 394
- EP-A- 1 704 827
- JP-A- 11 056 878
- US-A- 5 741 139
- US-A- 5 786 283
- US-A- 5 921 778
- US-A1- 2004 084 177
- US-A1- 2006 208 393
- US-B1- 6 200 136
- US-B2- 6 846 181
- US-B2- 7 235 290

## Description

The present invention relates to a semi-worked piece of composite material for producing dental/odontoiatric devices, such as posts and/or stumps and/or dental crowns, as well as to crowns and/or stumps and/or dental posts (or more generally, similar artefacts/devices) obtained from said semi-worked piece according to claim 1.

It is known that manufacture of posts/stumps for (endosseous or endodontic) dental implants, or more generally of mounting supports for dental/odontoiatric use, is based on employment of composite materials, i.e. materials comprising a polymeric matrix in which mechanically resistant fibres are nested.

Generally, these composite materials have elongated and continuous mechanically resistant fibres, which are disposed in conditions of mutual parallelism.

These semi-worked pieces, typically obtained from extrusion or pultrusion, are then submitted to turning and/or milling operations, so as in particular to make the so-called "posts" or "stumps".

At the same time, manufacture of artificial teeth (or, technically speaking, dental "crowns") makes use of substances having a "metallic" base and, among these substances, zirconium oxide is the one mainly employed. US 5921778 discloses a hybrid woven material for reinforcement of dental restorations. US 6846181 discloses a ready-to-use preshaped, prefabricated cured components for dental appliarices.US 7235290 discloses a prepeg containing fibers and a curable matrix used for dental applications.US 5741139 deals with dental post made of a plurality of fibers.

Although the known art briefly mentioned above is widely used, it however has some drawbacks.

First of all, the semi-worked pieces of composite material with parallel longitudinal fibres are subjected to structural decay if they are submitted to specific working operations, such as drilling along the extension axis of the reinforcing fibres; in fact, due to execution of these operations the composite material is submitted to excessive stresses along the transverse direction, which stresses in turn are due to the particular direction given to the reinforcing fibres; these stresses therefore cause structural yielding of the material which consequently is not suitable to be stably shaped (for instance, in posts of thin section and having an axial cavity).

In addition, while the zirconium oxide has an aesthetic/visual appearance greatly resembling that of a natural tooth, it has a mechanical behaviour characterised by a high hardness which, on the other hand, is combined with a high brittleness, and as a result, an artificial tooth made of this material is greatly subjected to breaking phenomena during its normal "use".

Moreover, while the zirconium oxide is biocompatible, it does not lend itself well to use for making dental crowns, because it has a surface greatly adapted to formation and retention of the dental plaque, so that its use gives rise to high risks of tartar deposits being accumulated.

A further drawback relating to the known art resides in that coupling of dental crowns of zirconium oxide is subjected to strong wear phenomena in the areas of mutual contact with the posts/stumps of composite material; this wear results from the high hardness of the contacting materials and is enhanced by the stresses to which the constraint area between crown and post/stump is subjected during mastication.

Generally, it is therefore to be noted that composite materials of known type to be used for making posts/stumps cannot be adapted to production of dental crowns and, vice versa, the metal-based materials used for making dental crowns cannot be used for producing posts/stumps.

Accordingly, the present invention aims at conceiving a semi-worked piece of composite material capable of obviating the above mentioned limits.

Mainly, the present invention aims at conceiving a semi-worked piece that can be indifferently used for making either dental crowns or posts/stumps, and that at the same time has an optimal combination of mechanical features (in terms of mechanical strength, resilience and the like) and biocompatibility features.

The present invention further aims at conceiving a semi-worked piece enabling an important operating flexibility in defining dental crowns, even of complicated shape; said dental crowns in turn should also have optimal duration, low or zero tendency to breaking or forming splinters and should also offer the most unfavourable environment to formation of dental plaque.

It is therefore an aim of the present invention to conceive a semi-worked piece that can be worked following different methodologies and along different intervention axes, without being submitted to losses of structural cohesion.

The technical task mentioned and the aims specified are substantially achieved by a semi-worked piece of composite material having the features reproduced in one or more of the appended claims.

Description of a preferred but not exclusive embodiment of a semi-worked piece of composite material in accordance with the invention is now given by way of non-limiting example and illustrated in the accompanying drawings, in which:

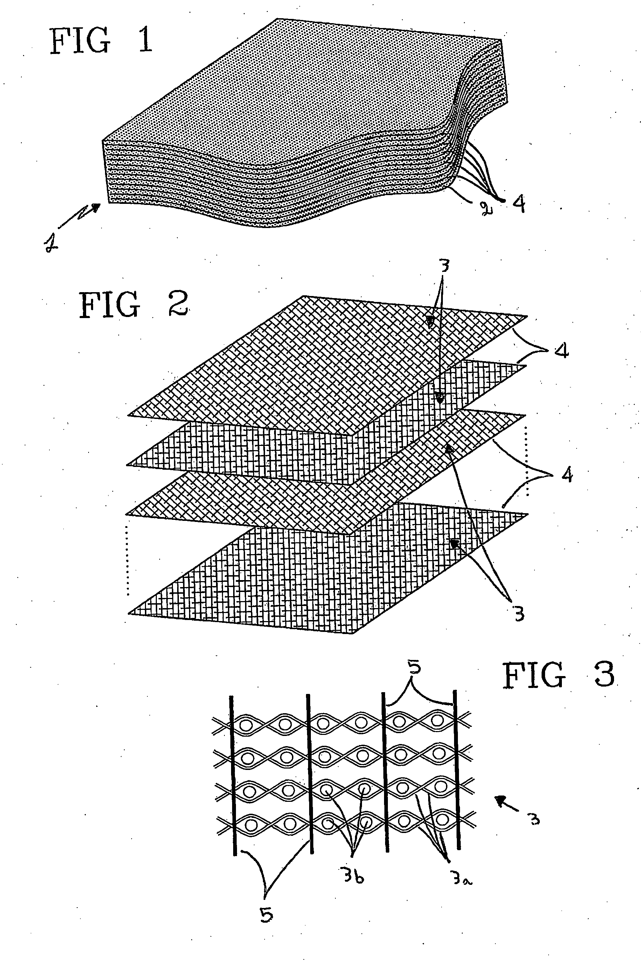

- Fig. 1 is a diagrammatic perspective view of a semi-worked piece in accordance with the invention;
- Fig. 2 is an exploded diagrammatic view of a pack of mechanically resistant fibres nested in the semi-worked piece seen in Fig. 1; and
- Fig. 3 is a side view in section of an alternative embodiment of the semi-worked piece in accordance with the invention.

With reference to the drawings, the semi-worked piece according to the present invention has been identified by reference numeral 1 and is substantially made of a so-called "composite material"; i.e. it comprises a polymeric matrix 2 and a multiplicity of mechanically resistant fibres 3 nested in this polymeric matrix 2.

Advantageously, in order to ensure an optimal mechanical behaviour of the "finished products" obtainable starting from the semi-worked piece (and at the same time in order to avoid creation of chipping or loss of structural cohesion between the reinforcing fibres during the operations for removing material from the semi-worked piece), the mechanically resistant fibres 3 are arranged in a predetermined number of substantially planar reinforcing layers 4 disposed in mutually superposed relationship.

In other words, the present invention is based on a particular arrangement of the reinforcing fibres inside the semi-worked piece, which arrangement ensures maintenance of the structural integrity should the semi-worked piece be submitted to working operations directed transversely/perpendicularly of the planar reinforcing layers 4; in fact, interknitting of the mechanically resistant fibres enables cohesion between fibres and polymeric matrix to be maintained around the work region where a forming tool operates (or, more generally, around the.penetration/advancing direction along which this forming tool is moving, which tool can be either a drill, a milling cutter or a device for material removal).

Maintenance of the structural integrity during working is therefore ensured by the topology/arrangement of the reinforcing fibres; in this regard it is to be noted that arrangement into superposed layers can be obtained in a great number of ways, such as through stacking of several textile materials of a predetermined (and preferably equal) area, or through subsequent folding of a single textile material the laps of which are therefore superposed upon each other without a break.

Likewise, depending on current requirements, interknitting of the resistant fibres (generally defined by a weft and a warp thread) may involve the presence of "open" or "closed" ends of the weft and/or warp threads; in this way it is possible to control the properties of the ends of the reinforcing layers depending on the "peripheral" finish degree of the semi-worked piece (or depending on the surface quality of the posts/stumps or of the dental crowns obtained from said semi-worked piece).

As above said, the reinforcing layers 4 comprise a multiplicity of weft threads 3a and warp threads 3b which are mutually interwoven according to at least one predetermined interknitting pattern; at the same time, arrangement in space of the reinforcing layers 4 contemplates definition of ideal lying surfaces that can be conveniently flat (so as to obtain the maximum manufacture simplicity of the semi-worked piece and the maximum homogeneity of the mechanical performance) or also extend into three-dimensional shapes.

From the point of view of the materials used, in the present semi-worked piece the resistant fibres 3 (or more generally the reinforcing layers 4) comprise glass fibres and/or aramidic fibres and/or carbon fibres and/or quartz fibres, while the polymeric matrix 2 comprises at least one epoxy resin and/or polyester and/or a material of the PEEK type or the like.

It should be recognised that selection of the materials for making the present semi-worked piece is mainly dictated by the final features that the posts/stumps and/or the dental crowns to be obtained from the semi-worked piece itself must possess; in particular, selection of the combination between mechanically resistant fibres and polymeric matrix, of the interknitting patterns of the reinforcing layers and others is carried out based on the optimal combination between mechanical resistance, elastic behaviour and surface hardness that is wished to be given to said manufactured products.

For the purpose of making manufactured products (either posts/stumps or dental crowns) having an exterior appearance as much as possible in compliance with the "organic" counterparts they have to replace, the polymeric matrix 2 can conveniently comprise coloured additives preferably of a shade adapted to repeat the colour of a true tooth (titanium oxide-based compounds, for example); the white/ivory colouring imparted by these additives can be advantageously utilised in order not to create contrast/semitransparency effects of the post/stump or in order to make the artificial dental crown "liable to be confused" with possible natural teeth placed adjacent thereto.

Should the current requirements need it (for instance in order to make posts/stumps or dental crowns having high mechanical resistance features) the present semi-worked piece may comprise transverse reinforcing elements 5 which are adapted to bear mechanical stresses transverse to the "lying surfaces" of the reinforcing layers 4; simultaneously, these reinforcing transverse elements 5 can be adapted to mechanically connect at least two reinforcing layers 4 that are mutually adjacent and/or superposed.

In an exemplary embodiment of the present invention said transverse reinforcing elements 5 comprise threadlike elements made of glass and/or aramidic and/or carbon fibre; these elements can be interknitted with the reinforcing layers according to different spatial arrangements, and can also consist either of "open" thread segments or of a single thread interknitted in a continuous and repeated manner through two or more reinforcing layers.

Consistently with the above description (and with the following claims), the present invention also relates to several devices for dental/odontoiatric use which are made (by turning, milling or more generally material removal) starting from a semi-worked piece in accordance with the invention.

For instance, starting from the semi-worked piece 1 it is possible to obtain a so-called endosseous or endodontic "post/stump" which comprises a fitting portion (insertable in an organic tissue of a patient, that can be either a rest of dental root or directly a maxilla/mandible bone) and a support portion (connected to the fitting portion and adapted to receive a dental crown in engagement); alternatively, it is possible to obtain a so-called "dental crown" which on the contrary comprises a hooking portion (to be coupled to a corresponding support portion of a post/stump that in turn can be of the type described here above) and an operating portion (connected to the hooking portion and shaped like a tooth or a series of adjacent teeth).

It is to be noted that in addition to the two above mentioned examples of devices (or "finished products") the present invention can also concern other types of "finished products" such as endosseous or endodontic posts (i.e. rod-like posts with a smooth or threaded surface) that do not necessarily have an end having the shape of a "stump" or, symmetrically, "stumps" that are not provided with extensions acting as fitting posts (but that, for instance, can be fitted on posts also of known type).

Manufacture of the above listed finished products takes place through a process for manufacturing devices for dental/odontoiatric use which, in turn, comprises the following steps:
- first of all, providing a polymeric matrix 2;
- simultaneously, providing a multiplicity of reinforcing fibres 3;
- defining a semi-worked piece 1 by impregnating the reinforcing fibres 3 with the polymeric matrix 2; and
- working the semi-worked piece 1 carrying out milling and/or turning and/or material removal operations, so as to obtain one or more of the above mentioned devices for dental/odontoiatric use.

Advantageously, according to the above process the following sub-steps are to be carried out in the step of defining the semi-worked piece 1:
- making a predetermined number of reinforcing layers 4 by weaving (or interknitting) of the reinforcing fibres 3; and
- superposing said reinforcing layers 4 on respective lying surfaces (for instance, lying surfaces parallel to each other and brought into mutually stacked relationship).

Conveniently, definition of the semi-worked piece 1, and more particularly the above listed sub-steps (i.e. the sub-steps defining the topology/spatial arrangement of the reinforcing fibres 3) are put into practice before the step of impregnating the fibres 3 with the polymeric matrix 2, and possibly an operating sub-step may be present which consists in weaving and/or interknitting also the transverse reinforcing elements 5 with the reinforcing layers 4 (preferably, before the step of impregnating the mechanically resistant fibres with the polymeric matrix 2).

The invention achieves important advantages.

In fact, due to the particular construction architecture of the present semi-worked piece (and above all due to the arrangement of the interwoven and superposed reinforcing layers), it is possible to obtain an optimal mechanical resistance; in particular, a high capability of withstanding drilling operations can be obtained, which operations can be carried out without causing structural yielding within the material itself.

By virtue of the described structure, it is therefore possible to indifferently make, using the same type of composite material, either "integrated" posts/stumps (or also simple posts or simple stumps) or dental crowns; in addition, this widened application flexibility is further promoted by the fact that it is possible to select a wide variety of polymeric matrices, as well as to add different "additives" to these polymeric matrices (such as titanium oxide for example, to give the semi-worked piece a colour corresponding to that of natural teeth).

Finally, it should be appreciated that the present invention allows low manufacturing costs to be maintained both for the semi-worked piece (in addition to different formats for retail sale/distribution) and for the devices obtained from said semi-worked piece, without involving particular complications, modifications and/or adaptations also as far as machines of known type (lathes, milling cutters, numeric control machines associable with CAD/CAM software) are concerned, which is advantageous for the global production economy and for the final price of the dental/odontoiatric products.

## Claims

1. A semi-worked piece of composite material for producing posts and/or stumps and/or dental crowns, comprising:
- a polymeric matrix (2); and
- a multiplicity of mechanically resistant fibres (3) nested in said polymeric matrix (2),
**characterised in that** said resistant fibres (3) are arranged in a predetermined number of reinforcing layers (4) that are substantially planar and disposed in mutually overlapped relationship, said reinforcing layers (4) comprising a multiplicity of weft threads (3a) and warp threads (3b) mutually interwoven according to at least one predetermined interknitting pattern.

2. A semi-worked piece as claimed in claim 1, wherein said mechanically resistant fibres (3) and/or said reinforcing layers (4) comprise glass fibres or aramidic fibres or carbon fibres.

3. A semi-worked piece as claimed in claim 1, wherein the polymeric matrix (2) comprises at least one epoxy resin or polyester or a PEEK.

4. A semi-worked piece as claimed in claim 1, wherein the polymeric matrix (2) comprises colouring additives of a shade adapted to repeat a colour of a true tooth and more preferably comprising titanium oxide.

5. A semi-worked piece as claimed in claim 1, wherein also present are transverse reinforcing elements (5) adapted to withstand mechanical stresses transverse to the lying surfaces of the reinforcing layers (4) or adapted to mechanically connect at least two reinforcing layers (4) that are mutually adjacent or superposed.

6. A semi-worked piece as claimed in claim 5, wherein said transverse reinforcing elements (5) comprise threadlike elements made of glass fibre or aramidic fibre or carbon fibre or quartz fibre.

7. An endosseous or endodontic post/stump, comprising a fitting portion insertable in an organic tissue of a patient and a support portion connected to said fitting portion and adapted to receive a dental crown in engagement, **characterised in that** it is made starting from a semi-worked piece as claimed in claim 1.

8. A dental crown for dental and/or odontoiatric use, comprising:
- a hooking portion to be coupled to a corresponding support portion of a post/stump of the type as claimed in claim 7; and
- an operating portion connected to said hooking portion and shaped like a tooth or a series of adjacent teeth,
**characterised in that** it is made starting from a semi-worked piece as claimed in claim 1.

## Patentansprüche

1. Halbfertiges Werkstück aus Verbundwerkstoff für die Herstellung von Stiftaufbauten und/oder Stümpfen und/oder Zahnkronen, umfassend:
- eine Polymermatrize (2); und
- eine Vielzahl mechanisch beständiger Fasern (3), die in der besagten Polymermatrize (2) verschachtelt sind,
**dadurch gekennzeichnet, dass** die besagten, beständigen Fasern (3) in einer vorbestimmten Anzahl von Verstärkungsschichten (4) angeordnet sind, welche im Wesentlichen eben und in wechselseitig überlagertem Verhältnis angeordnet sind, wobei die besagten Verstärkungsschichten (4) eine Vielzahl von Schussfäden (3a) und Kettfäden (3b) umfassen, die wechselseitig gemäß wenigstens eines vorbestimmten Webmusters verwoben sind.

2. Halbfertiges Werkstück nach Anspruch 1, wobei die besagten beständigen Fasern (3) und/oder die besagten Verstärkungsschichten (4) Glasfasern oder Aramidfasern oder Carbonfasern sind.

3. Halbfertiges Werkstück nach Anspruch 1, wobei die Polymermatrize (2) wenigstens ein Epoxidharz oder Polyester oder ein PEEK umfasst.

4. Halbfertiges Werkstück nach Anspruch 1, wobei die Polymermatrize (2) Farbadditive eines Farbtons umfassen, der geeignet ist, eine Farbe eines echten Zahns zu reproduzieren und der vorzugsweise Titanoxid enthält.

5. Halbfertiges Werkstück nach Anspruch 1, wobei des Weiteren quer verlaufende Verstärkungselemente (5) vorhanden sind, welche geeignet sind, mechanischen Belastungen standzuhalten, die quer zu den Liegeflächen der Verstärkungsschichten (4) einwirken oder welche geeignet sind, wenigstens zwei nebeneinander oder übereinander liegende Verstärkungsschichten (4) zu verbinden.

6. Halbfertiges Werkstück nach Anspruch 5, wobei die besagten quer verlaufenden Verstärkungselemente (5) fadenförmige Elemente aus Glasfaser oder Aramidfaser oder Carbonfaser oder Quarzfaser umfassen.

7. Enossaler oder endodontischer Stiftaufbau/Stumpf, einen passenden Abschnitt umfassend, der in ein organisches Gewebe eines Patienten eingesetzt werden kann, und einen Halterungsabschnitt umfassend, der mit dem besagten, passenden Abschnitt verbunden und geeignet ist, eine Zahnkrone eingreifend aufzunehmen, **dadurch gekennzeichnet, dass** er ausgehend von einem halbfertigen Werkstück nach Anspruch 1 gefertigt ist.

8. Zahnkrone für den zahntechnischen und/oder zahnärztlichen Gebrauch, umfassend:
- einen einhakenden Abschnitt, der mit einem entsprechenden Halterungsabschnitt eines Stiftaufbaus/Stumpfes des Typs aus Anspruch 7 gekuppelt werden soll; und
- einen Funktionsabschnitt, der mit dem besagten, einhakenden Abschnitt verbunden ist und wie ein Zahn oder wie eine Reihe nebeneinanderliegender Zähne geformt ist,
**dadurch gekennzeichnet, dass** er ausgehend von einem halbfertigen Werkstück nach Anspruch 1 gefertigt ist.

## Revendications

1. Pièce semi-finie en matériau composite pour produire des pivots et/ou des moignons et ou des couronnes dentaires, comprenant :
- une matrice polymérique (2) ; et
- une multiplicité de fibres mécaniquement résistantes (3) imbriquées dans la dite matrice polymérique (2),
**caractérisée en ce que** lesdites fibres résistantes (3) sont disposées en un nombre prédéterminé de couches de renforcement (4) qui sont essentiellement planaires et disposées en une relation mutuellement superposée (4), lesdites couches de renforcement (4) comprenant une multiplicité de fils de trame (3a) et de fils de chaîne (3b) mutuellement entre-tissés selon au moins un modèle de tricotage.

2. Pièce semi-finie selon la revendication 1, dans laquelle lesdites fibres mécaniquement résistantes (3) et/ou lesdites couches de renforcement (4) comprennent des fibres de verre ou des fibres aramides ou des fibres de carbone.

3. Pièce semi-finie selon la revendication 1, dans laquelle la matrice polymérique (2) comprend au moins une résine époxy ou en polyester ou un PEEK.

4. Pièce semi-finie selon la revendication 1, dans laquelle la matrice polymérique (2) comprend des additifs colorants d'une nuance apte à reproduire une couleur d'une dent véritable et comprenant de préférence de l'oxyde de titane.

5. Pièce semi-finie selon la revendication 1, dans laquelle sont également présents des éléments de renforcements transversaux (5) aptes à résister aux contraintes mécaniques transversales par rapport aux surfaces de couchage des couches de renforcement (4) ou aptes à raccorder mécaniquement au moins deux couches de renforcement (4) qui sont réciproquement contiguës ou superposées.

6. Pièce semi-finie selon la revendication 5, dans laquelle lesdits éléments de renforcement transversaux (5) comprennent des éléments filiformes réalisés avec des fibres de verre ou des fibres aramides ou des fibres de carbone ou des fibres de quartz.

7. Pivot/moignon endo-osseux ou endodontique, comprenant une partie de montage pouvant s'insérer dans un tissu organique d'un patient et une partie de support raccordé à ladite partie de montage et apte à recevoir une couronne dentaire en engagement, **caractérisée en ce qu'**il est réalisé à partir d'une pièce semi-finie selon la revendication 1.

8. Couronne dentaire pour l'usage dentaire et/ou odontologique, comprenant :
- une partie d'accrochage à accoupler à une partie de support correspondante d'un pivot/moignon du type selon la revendication 7 ; et
- une partie opérationnelle raccordée à ladite portion d'accrochage et façonnée comme une dent ou une série de dents adjacentes,
**caractérisée en ce qu'**elle est réalisée à partir d'une pièce semi-finie selon la revendication 1.
